# EUROPEAN PATENT SPECIFICATION

(11) **EP 0 644 742 B1**
(45) Date of publication and mention of the grant of the patent: **08.12.1999**
(21) Application number: 92914239.6
(22) Date of filing: 12.06.1992
(51) Int. Cl.: A61B 17/36, G02B 5/02, A61N 5/06

(54) **DIFFUSOR TIP FOR AN OPTICAL FIBER**
DIFFUSIONSSPITZE FÜR EINE OPTISCHE FASER
TETES DE DIFFUSION POUR UNE FIBRE OPTIQUE

(43) Date of publication of application: 29.03.1995
(73) Proprietor: Miravant Systems, Inc., Santa Barbara, CA 93117 (US)
(72) Inventor: DOIRON, Daniel, R., Santa Ynez, CA 93460 (US); NARCISO, Hugh, L., Jr., Santa Barbara, CA 93109 (US); PASPA, Paul, Santa Barbara, CA (US)
(74) Representative: Lehmann, Klaus, Dipl.-Ing.
(86) International application number: US9205013
(87) International publication number: WO9325155

(56) References cited:
- EP-A- 0 188 273
- EP-A- 0 266 031
- EP-A- 0 386 241
- EP-A- 0 394 446
- EP-A- 0 424 272
- EP-A- 0 437 183
- EP-A- 0 439 629
- EP-A- 0 450 149
- EP-A- 0 453 092
- DE-A- 4 137 983
- US-A- 4 273 109
- US-A- 4 466 697
- US-A- 4 660 925

## Description

### BACKGROUND OF THE INVENTION

### 1. Field of the invention

This invention relates generally to a means for cylindrically diffusing energy from an optical wave guide, and more particularly to a cylindrical diffuser tip for an optical fiber or fiber bundle, according to the preamble of claim 1. The diffuser tip generally is useful for performing Photodynamic Therapy in the treatment of diseased tissue such as tumors, inducing hyperthermia or performing both percutaneous and intraoperative phototherapy of cardiovascular disease.

### 2. Description of the prior art

The description of the prior art is conveniently introduced by a brief description of Photodynamic Therapy (PDT), the therapeutic modality giving rise to the current invention.

Photodynamic treatment of tumors requires irradiation of the tumor under treatment with light of a wavelength that penetrates tissue, usually around 630-800 nanometer range, generally by means of a laser. A short time prior to irradiation, the patient is injected with an appropriate photo-sensitive compound which selectively localizes in a target tissue such as a hematoporphyrin derivative which accumulates in the vascular stoma of the tumor and in the tumor cells. Subcutaneous tumors greater than 1.0 cm thick, also referred to herein as interstitial tumors, undergoing this treatment require the use of optical fibers to guide the light from the source to the deeper treatment area. In many cases, the outlet termination of the fiber is inserted directly into the tumor. In other cases, where the tumor is located in passages, for example endobronchial tumors, the optical fiber termination is positioned intraluminally in juxtaposition to the tumor. Similarly, photo irradiation of pre-sensitized atheromatous plaque on the wall of a blood vessel , a procedure known as photoatherolytic therapy, has been shown to be an effective method for removing such plaque. Accordingly suitable fiber terminations for the delivery of a uniform, predictable dosage of light irradiation to a large volume of tumor tissue or the wall of a tubular tissue are needed.

Considerable light radiation must be transmitted to kill large tumor masses by photo irradiation and the required radiation can cause undesired overheating, especially if it is concentrated in too small a region. This causes problems in delivering radiant energy out of the end of a normal blunt ended or flat cut optical fiber, making a small hot spot which may lead to excessive heating, carbonization and necrosis of the adjacent tissue rendering such tissue opaque to transillumination. Thermal sources such as xenon arc lamps also pose difficulties in transmitting adequate radiation to deep-seated tumors because non-coherent sources cannot be coupled efficiently to reasonably small optical fibers for delivery to the tumor. Problems of distributing radiation uniformly throughout the region of a tumor to be killed are also formidable.

An optical fiber generally consists of an optically transmissive core, a reflective cladding surrounding the core and an outer jacket which facilitates handling. There are several ways that the cylindrical diffusion of radiant energy from an optical fiber core can be accomplished. One way is to choose a ratio of the indices of refraction between the cladding and the core region of the optical fiber so that internal reflection within the core region is substantially less than total. This causes light to enter the cladding. If scattering centers are present in the cladding the light can radiate outward to emerge through the (preferably transparent) cladding.

Another way is to alter the interface between the optical fiber core and cladding to increase side radiation. Texturing the outer surface of the core region to provide a ground glass effect is one method commonly used. Another is positioning or embedding light scattering elements such as tiny particles at the surface of the optical fiber core near the interface with the cladding. Light scattering particles can also be imbedded throughout the cladding to enhance the side delivery of radiation. Combinations of these measures are also possible.

For example, Chapman in United Kingdom patent GB2154761A (issued 11 September 1985) describes an optical fiber for use in Photodynamic Therapy wherein the fiber comprises a central core material enveloped by a special two-layer cladding. The cladding comprises an inner cladding of a low refractive index material and an outer cladding. The fiber, one end of which is adapted to be coupled to a laser beam, has an output end portion which has a tapered core region which is surrounded by a diffusing medium. Light emerging from the tapered core region undergoes scattering in the diffusing medium.

In one preferred embodiment, Chapman's core is of circular cross section and the diameter of the core in the tapered region decreases uniformly to an end most point over a length of between 5 and 15 millimeters. In a further preferred embodiment, Chapman describes a diffusion medium comprising a transparent resin material, which contains fine particulate reflective or refractive matter. All embodiments require removing the cladding from around the core thereby weakening the fiber.

Clark, in U.S. Patent No. 4,336,809 describes a tissue photo irradiation system for use with hematoporphyrin dyes and derivatives thereof. Clark describes the use of an optical needle which serves as a linear radiator or a cylindrical diffuser and which can be coupled to an optical fiber by means of a conventional optical coupler. Clark's needle includes a fiber optic core that is generally internally reflecting. The core is surrounded by a cladding as generally known; but in an end region a different cladding surrounds the core to make it into a radiator instead of an internally reflecting transmitter. When the cladding contains scatterers, the "needle" or diffusion tip comprises a transparent core surrounded by a scattering layer in which the concentration of scatterers is homogeneous along its length.

McCaughan, Jr., in U.S. Patent 4,660,925, describes a cylindrical diffuser tip that overcomes some of the problems with prior art diffuser tips. McCaughan Jr. suggests (column 4, lines 48-62) providing a tip surrounding the core of an optical fiber, the tip containing a gradient of scatterers which increases logarithmically in concentration along the fiber axis in a direction toward the polished tip of the optical fiber. To accomplish this, McCaughan, Jr. teaches a method for making such a tip comprising the steps of exposing (i.e. stripping the cladding away from) the core of an optical fiber near its tip, polishing the exposed core and repeatedly dipping the tip in a curable medium containing different concentrations of scatterers to allegedly increase the concentration of scatterer along the length of the exposed core. The polished tip of the core (column 5, lines 47-49) region is cleaned of scattering medium upon removal from the dipping vessel. The word "allegedly" is used above because such a method of repetitive coating followed by the step underlined above is inoperable to produce a longitudinal gradient of scatterer in a diffuser tip. This method produces a radial gradient in scatterer concentration which varies radially with distance from the fiber core axis. Even if this method could, by further experimentation, be made operable, such a method would provide, at best, a discrete, step-wise concentration gradient which would only approximate a logarithmic gradient in the limit of infinite coatings.

Notwithstanding the inoperability of the McCaughan disclosure for making a diffuser tip with the desired properties, the tip, as with all prior art diffuser tips, requires the stripping of the cladding and jacket surrounding the optical fiber core and replacing the cladding with a material in which scattering centers are embedded. Such a construction weakens the optical fiber where the cladding has been removed rendering it vulnerable to breakage.

Production of a controllable level of temperature elevation or hyperthermia at pre-selected locations in a target tissue has been found to be of significant therapeutic value in the treatment of patients with cancer. In particular, hyperthermia may, in some cases, have a synergistic effect when used in conjunction with Photodynamic Therapy for treating tumors or performing angioplasty. The terms "selective" and "non-selective" as commonly applied to hyperthermia in the literature refers to tissue temperatures under 45 °C. and over 45 °C. respectively. The term "hyperthermia" as used herein, refers to any increase in tissue temperature above normal. At the high power levels required for hyperthermia or hyperthermia plus Photodynamic Therapy, high peak intensities or hot spots can lead to excessively high temperatures causing unintentional non-selective tissue damage. It is, therefore, desirable to distribute the illuminating energy evenly within the target volume to achieve uniform temperature distributions.

The present optical fiber cylindrical diffuser tip technology is limited in clinical applications due to the following:
a) The underlying optical fiber is weakened by mechanical processing during manufacturing of the cylindrical diffusing tip, particularly by removal of any portion of the protective cladding from around the fiber core;
b) The weakened fiber optic limits the flexibility of the finished cylinder diffuser rendering it suitable only for quasi-rigid usage (very limited endoscopic use);
c) Light must be coupled into an optical fiber for delivery to tissue. The variation in output of prior art cylindrical diffuser tips with variation in input beam convergence causes extreme variability in the output intensity distribution.
d) A non-uniform output intensity distribution makes treatment dosimetry uncertain and clinical results inconsistent;

It is desirable, therefore, to provide a cylindrical diffuser for use as a termination on an optical fiber which overcomes most or all of the limitations stated above. In particular, it is desirable to provide a cylindrical diffuser tip for an optical fiber that does not require removal of the cladding surrounding the optical fiber core thereby improving durability of the fiber.

Further, a diffuser tip for an optical fiber is known from EP-A-0 437 181 which forms the preamble of claim 1. Therein, an apparatus for illuminating certain internal portions of a patient for photodynamic therapy with an optical fiber is disclosed, the apparatus having a diffuser tip for said optical fiber. This known device comprises an optical fiber having a core and a cladding overlying said core, and an outer sheath is provided overlying said cladding. Further, this device comprises a diffuser tip having a core which has a diameter equal to or greater than the diameter of the optical fiber cladding. A layer surrounds the core of the diffuser tip and the sheath of the optical fiber. This known device is intended to deliver uniformly cylindrical illumination to the interior wall of a tubular tissue such as the bronchi or the esophagus.

Still another diffuser tip for an optical fiber is disclosed in EP-A-0 450 149, wherein the core of an optical fiber at its end is coated with a layer of scattering medium. This arrangement is enclosed by a sleeve, leaving a free space between the sleeve and the layer. However, this diffuser tip does not have a central core separate from the core of the optical fiber. Rather, here the core of the diffuser tip is formed integrally with the core of the optical fiber. This device is a cylindrical fiber optic diffuser for use in photodynamic therapy dispersing light outwardly in a cylindrical scattering pattern.

Therefore, it is the object of the invention to provide an alternative to the above mentioned devices.

One object according to a preferred embodiment of this invention is to provide a diffusion tip for an optical fiber, the tip having a continuous concentration gradient of scattering centers throughout its length which enables the cylindrical diffusion of radiant energy from the fiber uniformly along the length of the tip.

Still another object according to a preferred embodiment of this invention is to provide a cylindrical diffuser tip for an optical fiber which retains the structural integrity of the fiber and does not require removing the cladding from the fiber for insertion thereon.

Another object according to a preferred embodiment this invention is to provide a cylindrical diffusion tip for an optical fiber which is flexible.

Yet a further object according to a preferred embodiment of this invention is to provide a diffuser tip for an optical fiber which tip avoids high intensity non-uniform distribution of light energy commonly referred to as "hot spots" caused by silica fiber core manipulation or discrete scatterer concentration variations.

Yet a further object according to a preferred embodiment is to provide an optical fiber cylindrical diffusion tip for use in Photodynamic Therapy treatment of tumors that efficiently and uniformly cylindrically diffuses transmitted light and which diffuser tip has a maximum diameter substantially the same as that of the fiber incorporated therein.

Still another object according to a preferred embodiment of this invention is to provide an optical fiber diffuser tip which is useful for the treatment of atheromas in angioplasty or photoatherolytic therapy.

Yet another object according to a preferred embodiment of this invention is to provide a terminal diffuser tip for an optical fiber useful for inducing hyperthermia in selected target tissue.

Another object according to a preferred embodiment of this invention is to provide a diffuser tip for an optical fiber which has a variable composition that may be altered to provide a customized distribution of diffused light from the diffuser tip.

A further advantage of the termination tip according to a preferred embodiment of the present invention is that it is input mode independent. That is, the distribution of light out of the diffuser is independent of the coupling mode.

### SUMMARY OF THE INVENTION

The above mentioned problem is solved by a diffuser tip for an optical fiber according to claim 1.

Preferred and advantageous embodiments of the invention are given in the dependent claims. Accordingly, the diffuser tip has a continuous concentration gradient of scattering centers throughout its length which enables the cylindrical diffusion of radiant energy from the fiber uniformly along the length of the tip. Further, the diffuser tip has a variable composition that may be altered to provide a customized distribution of diffused light.

Several embodiments of the present invention teach the use of a diffusion tip in which a portion of the outer jacket of a conventional optical fiber is stripped away near the tip to expose the cladding surrounding the core. The cladding remains covering the core for protection against breakage. The optical fiber tip is terminated by abutting the exposed tip of the fiber with an elastomeric diffuser tip core, the outer diameter of the diffuser tip core being slightly greater than that of the optical fiber's core yet less than or equal to the outer diameter of the optical fiber's jacket. In one embodiment, the diffuser tip core comprises a continuous gradient of scattering centers embedded in an optically transparent elastomer substrate comprising preferably transparent silicone wherein the light scattering centers continuously increase in concentration along the diffuser tip core axis in a direction away from the optical fiber face. In other embodiments, the increase in scatterer concentration in the diffuser tip core may be stepwise or continuous. In one embodiment, an air space is disposed between the tip of the optical fiber core and the core of the diffuser tip. This geometry allows the use of higher power by coupling substantially the same amount of power as the directly contacting embodiments (Figs. 2-5) to the diffuser tip while reducing the power density considerably. Each of the diffuser tips described below produces a predictable and repeatable output distribution pattern which can be tailored to any application. All of the embodiments retain the cladding surrounding the core of the optical fiber along the entire length of the optical fiber core.

### BRIEF DESCRIPTION OF THE FIGURES

Figure 1 is a schematic cutaway view of one embodiment of a diffuser tip of the present invention.
Figure 2 is a cutaway schematic view of an embodiment of a diffusion tip which is pointed to facilitate insertion through tissue.
Figure 3 shows a cutaway schematic view of a further embodiment of a diffusion tip which is beveled to facilitate interstitial illumination or surgery.
Figure 4 is an isometric close-up view of the cylindrical diffuser tip of Figure 3.
Figure 5 is a cutaway side view of the diffuser tip of Figure 3 in which the silicone tip is molded onto the optical fiber in three stages; each stage having a greater concentration of scatterer than the previous one.
Figure 6 is similar to Figure 5 except that the silicone diffuser tip comprises three separate silicone plugs each having a different concentration of scatterer and not molded to the fiber.
Figure 7 is a cutaway side view of a diffuser tip of the present invention in which the silicone tip abutting the end face of the optical fiber is tapered.
Figure 8 is a schematic view of a multi-channel single head extruder useful for making an extruded diffuser tip core having a continuous controllable gradient of scatterers along its length.
Figure 9 is a longitudinal cross-sectional view of an embodiment of a continuous gradient diffusion tip with the tip pointed to facilitate insertion into tissue.
Figure 10 shows a longitudinal cross-sectional view of another embodiment of a diffusion tip having a continuous distribution of scattering centers and a rounded tip useful for intraluminal illumination of tubular tissue.
Figure 11 is a longitudinal cross-sectional view of an embodiment of a diffuser tip in which the silicone tip is molded onto the optical fiber in two stages; one stage adding a conical transparent silicone core, the second stage adding a surrounding core containing scatterers arranged in a continuous gradient along the length of the diffuser.
Figure 12 is similar to Figure 9 except that the continuous gradient silicone diffuser tip is separated from the tip of the optical fiber by a space which may contain air, gas, or liquid for high power application.
Figure 13 shows a longitudinal cross-sectional view of a continuous gradient cylindrical diffuser tip having a guidewire compatible lumen forming a central axis for use in intraluminal applications such as angioplasty.
Figure 14 shows the insensitivity of the distribution of light emanating from the fiber optic diffuser tip according to the present invention for two different modes of coupling light into the optical fiber.
Figure 15 is a schematic diagram of a light delivery catheter.
Figure 16 is a cross-section of the light delivery catheter of Figure 15 along line 16-16.
Figure 17 is a cross-sectional view of the light delivery catheter of Figure 15 along line 17-17.
Figure 18 is an end view of the light delivery catheter of Figure 15 along line 18-18.
Figure 19 is a plan view of the distal tip of the light delivery catheter of Figure 15.
Figure 20 is a cross-section of the distal tip of the light delivery catheter of Figure 19 taken along line 20-20.
Figure 21 is a longitudinal cross-section of the distal tip of the light delivery catheter of Figure 19 taken along line 21-21.
Figure 22 is a cutaway view of a tubular tissue into which tissue the guidewire compatible diffuser tip-bearing catheter of Figure 21 has been inserted.

### DESCRIPTION OF THE PREFERRED EMBODIMENTS

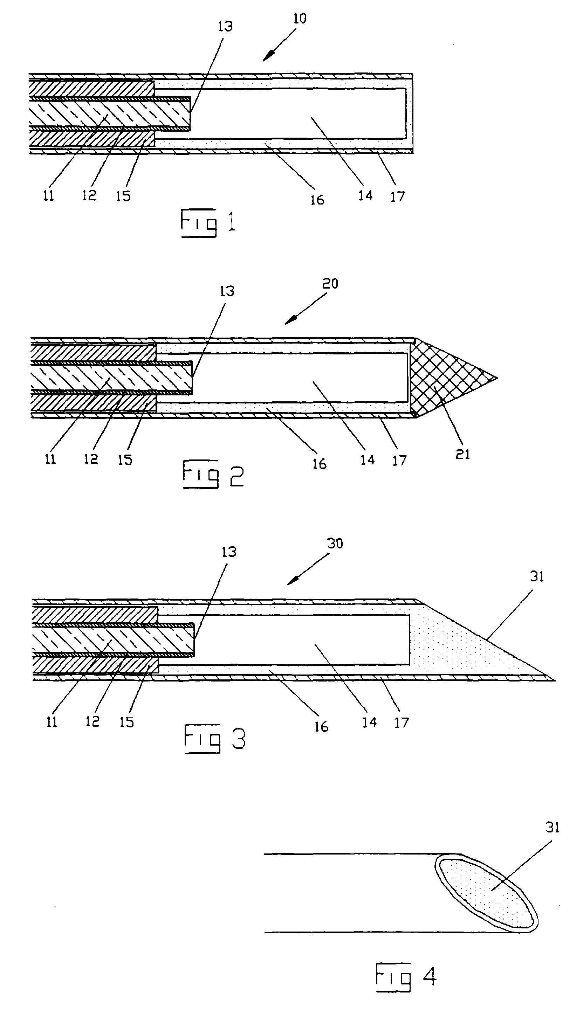

A preferred embodiment of the optical fiber cylindrical diffuser tip of the present invention is indicated generally at 10 in Figure 1. A standard optical fiber consisting of a core 11 and cladding 12 has a blunt distal end indicated at 13. A silicone tip 14 has a proximal end which is recessed to accommodate the distal end 13 of optical fiber core 11. In practice, the outer diameter of the silicone tip is greater than the outer diameter of the optical fiber core 11 plus cladding 12 but less than the outer diameter of the jacket 15. The silicone tip 14 may be any length but is preferably between 0.5 and 5 cm for Photodynamic Therapy irradiation of tumors.

After the silicone tip 14 is affixed to the optical fiber core, a second layer 16 consisting of silicone containing a scatterer is coated over the silicone tip by injection molding or dipping the silicone fiber into a suspension containing a scatterer such as diamond dust, Ti 02 or alumina in uncured silicone. The scattering layer 16 may then be vulcanized to the silicone tip by curing at an elevated temperature as is well known in the art. The diffuser tip thus obtained is, however, extremely flexible and, in practice, it is desirable to stiffen the fiber by the insertion of an optically transparent tubing 17 over the silicone tip.

The choice of material and wall thickness of the tubing 17 will ultimately determine the flexibility of the diffuser tip. The distribution of scattered light emanating from the silicone diffuser tip can be controlled by varying the concentration of scatterer in the silicone. If the concentration of scatterer is homogeneous along the length of the diffuser tip, a linear intensity distribution results.

Turning now to Figure 2, a diffuser tip, generally indicated at 20, for use in irradiating interstitial tumors, is similar to the diffuser tip 10 but has a sharp pointed tip 21 enabling the tip to be pushed into and through tissue with or without the aid of a cannula. For interstitial use, it is desirable that the tip have as small an outer diameter as possible to facilitate penetration to the tumor. This interstitial cylindrical diffusion tip is concurrently made by bonding the silicone tip 14 to the optical fiber core 11 and cladding 12. Once cured, the tubular tip jacket 17 may be slipped over the core jacket 15 and pushed up the fiber beyond the optical fiber's distal end 13. The scattering layer 16 may then be applied as before and while the uncured silicone layer is still wet, the tip jacket 17 sleeve is pulled toward the tip 21 of the diffuser until it projects past the distal end 14 tip of the silicone tip. A conical translucent or opaque piercing tip 21 is then pressed into the uncured scattering layer and the assembly is allowed to cure.

It is important that the silicone used in both the fiber and the scattering layer be free of bubbles. This may be facilitated by applying a vacuum to the uncured silicone prior to use. Optical quality silicone, available from McGhan NuSil Corporation, Carpinteria, California, U.S.A. Catalog No. CF1-6755 is suitable for the construction of such tips.

A further advantage of using a tip jacket 17 with the flexible silicone tip and scattering layer is the ability to shape the distal end of the tip jacket 17 into a piercing or cutting tool suitable for insertion into tissue or for performing laser surgery. Figure 3 shows such an interstitial cylindrical diffuser generally indicated at 30 wherein the tip jacket 17 and scattering layer 16 of the diffuser 31 has been beveled to form a cutting blade which is shown in greater detail in Figure 4. The sharp tip 31 can be used to cut through tissue.

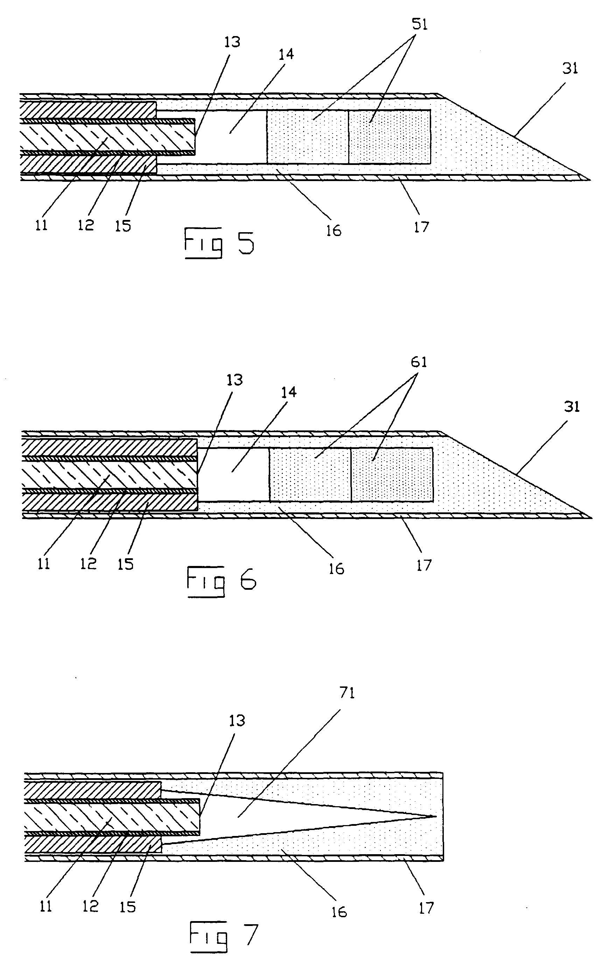

Turning now to Figure 5 and Figure 6, further preferred embodiments of the silicone diffuser tip of the present invention are shown in which the scattering centers are incorporated into the silicone tip 51 as well as in the scattering layer 16. In Figure 5, the inner silicone tip is molded onto the optical fiber in three stages; each stage with a greater concentration of scatterer than the previous one. A stepwise concentration gradient of scattering centers is thereby achieved which may be varied to customize the intensity distribution of the diffused light for particular applications. The number of stages may be made greater if desired to achieve a smooth, nearly continuous variation in the concentration of scatterer along the tip. Alternatively, as shown in Figure 6, silicone plugs 61 containing various concentrations of a suitable scatterer may be molded within the tubular scattering layer 16 to form a diffuser tip which tip may then be inserted over the distal end 13 of the optical fiber core 11 and cladding 12 and held firmly in place against the end of the optical fiber by tip jacket 17.

Figure 7 shows yet another preferred embodiment of the present invention in which the silicone tip 71 is tapered. As the optically transparent central core of the silicone tip decreases in diameter, the exiting laser light encounters a greater number of scatterers in the scattering layer 16 before exiting the tip jacket 17. The intensity distribution of the cylindrically diffused light will be smoother than with the stepwise concentration gradients encountered by the light as it enters the plugs containing various concentrations of scatterers as shown in Figures 5 and 6.

As mentioned earlier, sensitivity of the output of prior art cylindrical diffuser tips to input beam divergence causes extreme variability in the output intensity distribution. The distribution of light emanating from the various embodiments of the optical fiber diffuser tips of the present invention is substantially independent of the coupling mode of the light source into the optical fiber or fiber bundle.

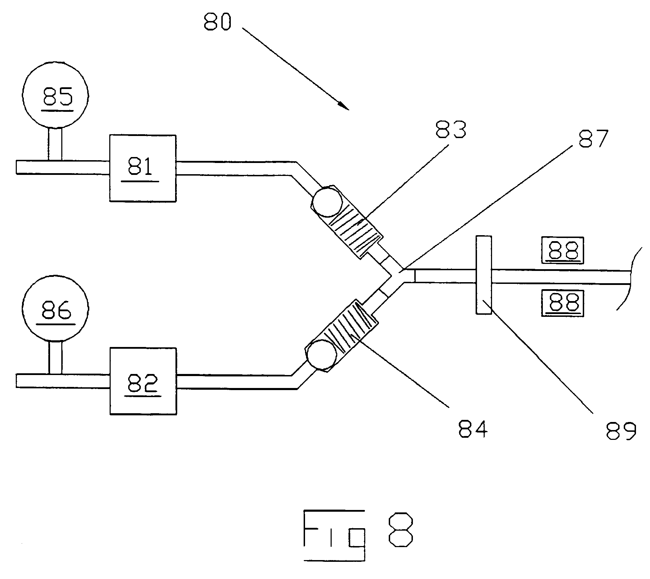

A diffusion tip having a continuous gradient of scattering centers embedded in an optically transparent extrudable material such as silicone elastomer is conveniently made by the extrusion process. Extrusion processes for forming cylindrical articles are well known in the art. Extruders are also commonly used to produce tubing as shown, for example, in U.S. patent 4,053,274. An extrusion apparatus adapted to produce a concentration gradient of scattering centers in the elastomer substrate is shown in Figure 8. To produce an elastomeric core for the tip having the desired gradient (which may or may not be linear) of light scatterers embedded therein, a dual injector system (or multiple injector system) generally indicated at numeral 80 in Figure 8 is employed. A first injector 81 contains a mixture of highly concentrated scatterers in the elastomer base. A second injector 62 contains a low concentration of scatterers in elastomer or elastomer alone. The two mixtures are forced through check values 83 and 84 respectively by pumps 85 and 86 and into a mixer 87 where they are combined in volumes predetermined to produce the desired final concentration of scatterers in the core 94 which emerges from the extruder 80 through one or more orifices (dotted lines) of an extrusion die 89. It is preferable to have the volume of the mixer 87 less than or equal to the volume of the orifice(s) in the extruder die 89 to enable greater control over the concentration of light scattering centers in the extruded fiber.

While the dual injection and mixing processes are occurring, an on-line detection system 88 monitors the concentration of light scatterers in the core as the core 94 is extruded. This information is relayed to a controller system (not shown) which regulates the flow from each injector to produce the optimum concentration gradient. Once determined, this optimum concentration gradient can be reproduced by programming the appropriate algorithms into a controller which independently regulates injectors 81 and 82.

The extruded core 94 comprising an extrudable transparent elastomer with the desired concentration gradient of scattering centers embedded therein is cured and forced into a plastic tube, the inner diameter of the tube being equal to or greater than the outer diameter of the optical fiber to which the tip is to be attached.

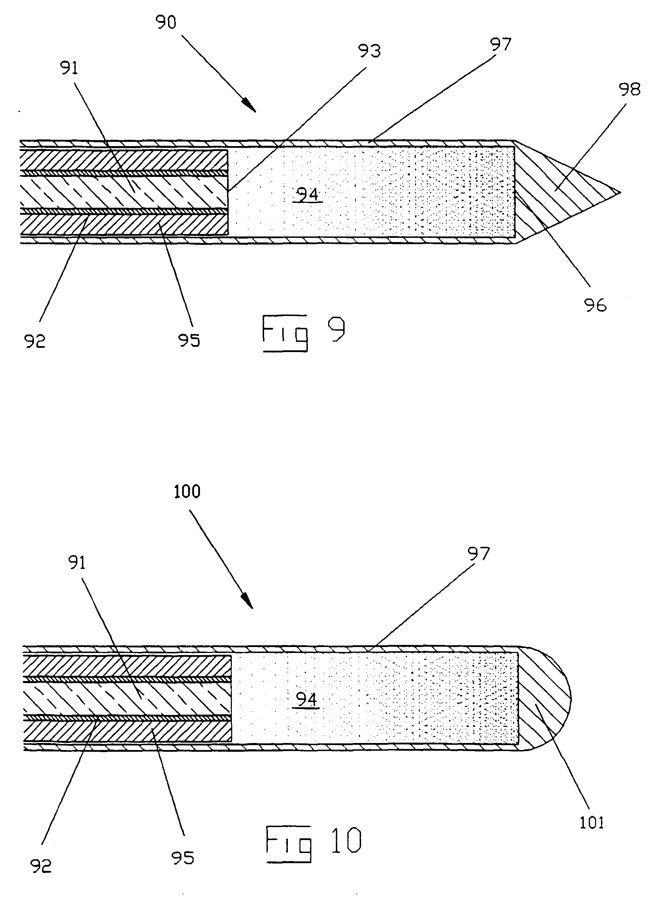

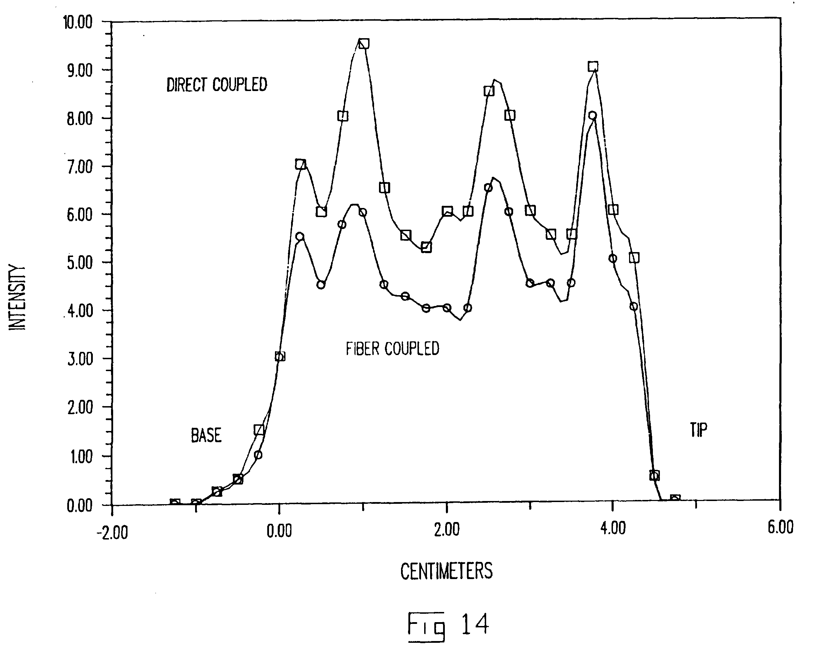

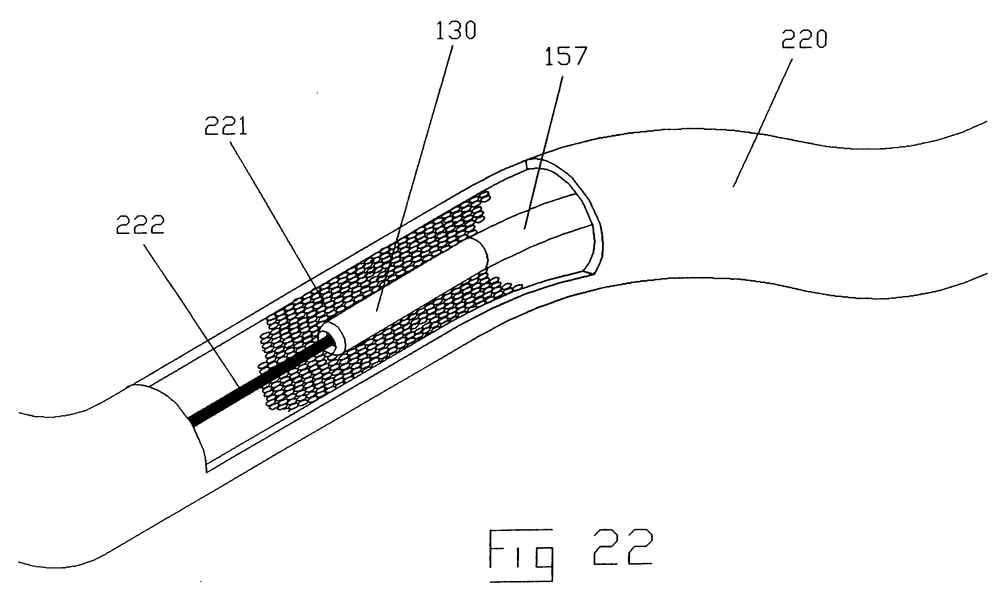

In the following, further embodiments of an optical fiber cylindrical diffuser tip are described in conjunction with Figs. 9 to 13 and 15 to 22. However, these embodiments do not represent the invention as defined in claim 1 and thus are not claimed. Fig. 14 and its related description explain the intensity of light distributed from the optical fiber diffuser tip of the invention.

A first such embodiment of the optical fiber diffuser tip is indicated generally at 90 in Figure 9. A standard optical fiber consisting of a core 91, cladding 92 and jacket 95 has a blunt distal end indicated at 93. The core of the diffuser tip 94 is preferably extruded from transparent silicone and has a proximal end which is recessed to accommodate the distal end 93 of an optical fiber and a distal end 96. In practice, the outer diameter of the core (94) of the cylindrical diffuser tip 90 is greater than the outer diameter of the optical fiber core 91 plus cladding 92 plus jacket 95. The diffuser tip 90 may be any length but is preferably between 0.5 and 5 cm for Photodynamic Therapy irradiation of tumors, phototherapy of cardiovascular disease, and laser induced hyperthermia of tumors.

After the silicone core 94 of the diffuser tip 90 is affixed to the optical fiber terminus 93 a plastic tube 97 is slid over the diffuser tip core 94 and optical fiber jacket 95 to provide a secure bond therebetween. The diffuser tip 90 thus obtained may be extremely flexible without the plastic tube 97. In practice, it is desirable to stiffen the fiber by the insertion of an optically transparent tubing 97 over the silicone core of the diffuser tip. A pointed tip 98 may be affixed to the tube 97 to facilitate interstitial insertion.

The choice of material and wall thickness of the tubing 97 will ultimately determine the flexibility of the diffuser tip. The distribution of scattered light emanating from the silicone core of the diffuser tip can be controlled by varying the concentration gradient of scatterer in the silicone. If the concentration of scatterer is moderate and homogeneous along the length of the core of the diffuser tip, a linear intensity distribution with negative slope results. If the concentration increases exponentially along the core the distribution of radiant energy emanating radially from the core will be linear with a zero slope as is desired. Other gradients can, of course, be used to generate other desired distributions of light.

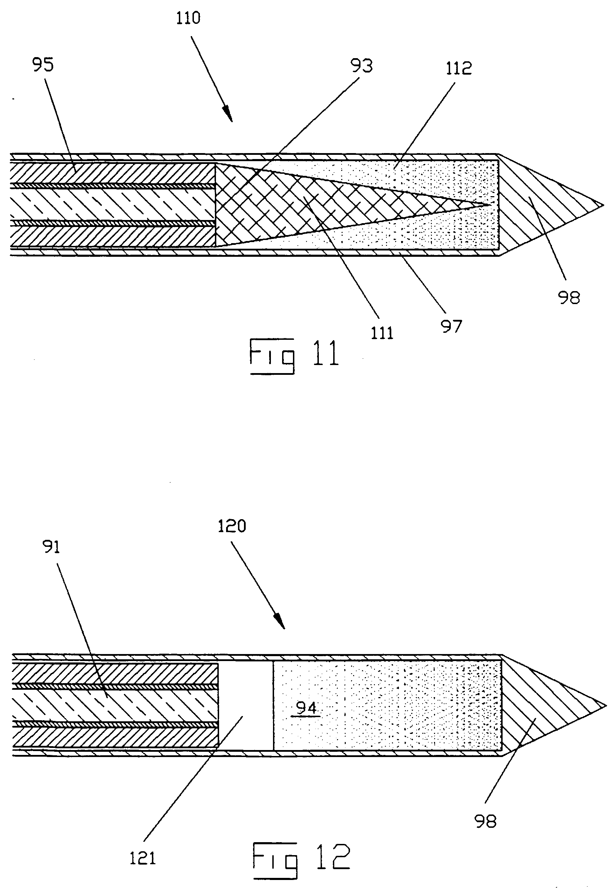

Figure 10 shows a cylindrical diffuser tip 100 in which the terminal tip 101 of the diffuser is rounded to enable insertion into the lumen of tubular tissue without the danger of puncturing the wall of such tissue. This tip is useful, for example, for illuminating tissues along the wall of the esophagus, bronchi, urethra or blood vessels.

Turning now to Figure 11 a diffuser tip, generally indicated at 110, for use in irradiating interstitial tumors, is similar to the diffuser tip 90 in that it has a sharp pointed tip 98 enabling the tip to be pushed into and through tissue with or without the aid of a cannula. For interstitial use, it is desirable that the tip have as small an outer diameter as possible to facilitate penetration of the tumor. This interstitial cylindrical diffusion tip has a core consisting of a transparent conical portion 111 and a scattering portion 112. The tip 110 is made by first bonding the conical portion 111 of the core to the optical fiber terminus 94. Core scattering portion 112 is made by extruding core 94 (not indicated in Fig. 11) as described earlier and removing a conical section therefrom to accommodate the conical portion 111. Scattering section 112 is then bonded to transparent conical portion 111 and allowed to cure. Once cured, the tubular tip jacket 97 may be slipped over the core 112 and fiber jacket 95 and pushed up the fiber beyond the optical fiber's terminus 93. The conical translucent or opaque piercing tip 98 is then pressed into the uncured scattering layer and the assembly is allowed to cure. This tip is particularly useful for inducing hyperthermia in tissue surrounding the tip.

Figure 12 shows yet another cylindrical diffuser tip, indicated at numeral 120 for irradiating interstitially. In this embodiment, a gap 121 between the fiber optic terminus and the proximal end of the silicone/scatterer core 94 of the diffuser tip 120 is incorporated. The gap 121 may be filled with air, gas, or liquid. The gap 121 provides space for a coupling fluid (gas or liquid) which reduces light energy density at the elastomer from the fiber optic by allowing the output to expand substantially prior to coupling to the core 94, thus the overall energy remains virtually unchanged. This configuration 120 allows for much higher power handling capabilities.

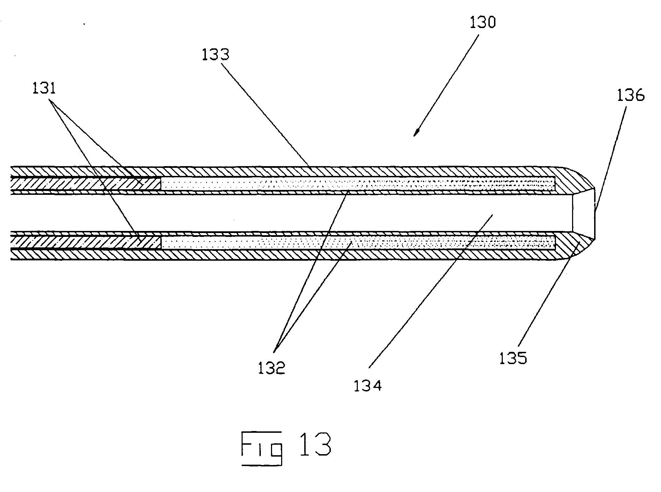

Figure 13 shows a continuous gradient diffuser tip adapted for use with a guidewire-compatible angioplasty catheter or the like for irradiating target tissue on the wall of tubular tissue. The diffuser tip 130 has a proximal end which abuts against the annular array of fiber optics 131 of an angioplasty catheter and a distal end 135. The annular core 131 of the catheter usually consists of a tubular bundle of optical fibers disposed around a central lumen 134. The central lumen 134 of the catheter is dimensioned to accommodate a guidewire therewithin. The core 132 of the diffuser tip is extruded in the manner described earlier except that a mandrel (not shown) is disposed centrally within the orifice of the extruder die 89 (Figure 8) in a manner well known in the art for extruding tubular members. The diffuser tip core 132 formed and described above has a continuous gradient of light scattering centers embedded therein which increase continuously in concentration from the proximal end to the distal end. The diffuser tip 130 may be inserted over a guidewire (not shown) by means of the guidewire portal 136.

It is important that the silicone used in the diffuser tip core be free of bubbles. This may be facilitated by applying a vacuum to the uncured silicone prior to use. Optical quality silicone, available from McGhan NuSil Corporation, Carpinteria, California, U.S.A. (Catalog No. CF1-6755) is suitable for the construction of such tips.

As mentioned earlier, sensitivity of the output of prior art cylindrical diffuser tips to input beam divergence causes extreme variability in the output intensity distribution. The distribution of light emanating from each of the embodiments of the diffuser tip of the present invention is substantially independent of input coupling mode as shown in Figure 14.

The light delivery system described herein is a highly flexible light-conducting catheter designed to be used over a guidewire for safety. The guidewire is intended to lead the catheter through tortuous and constricted pathways such as arteries. For this reason, flexibility of the catheter is important. The catheter must be flexible enough to track the guidewire along a serpentine route. A stiff catheter would tend to lead the guidewire with resulting perforation of the arterial wall. A dedicated guidewire lumen is, therefore, incorporated into this delivery system to house the guidewire. This allows the guidewire to remain in place during positioning and treatment. The treatment, or light conducting fibers never come in contact with the guidewire.

The catheter is also adaptable for use with a fluorescence probe. A fluorescence probe may be used to detect either endogenous fluorescent molecules in target tissue or elevated levels of exogenous chromophores such as hematorporphyrin derivative (HpD), dihematoporphyrin ether (DHE), or tin etiopurpurin (SnET₂). For exogenous chromophores such as HpD the excitation wavelengths employed is 630 nanometers.

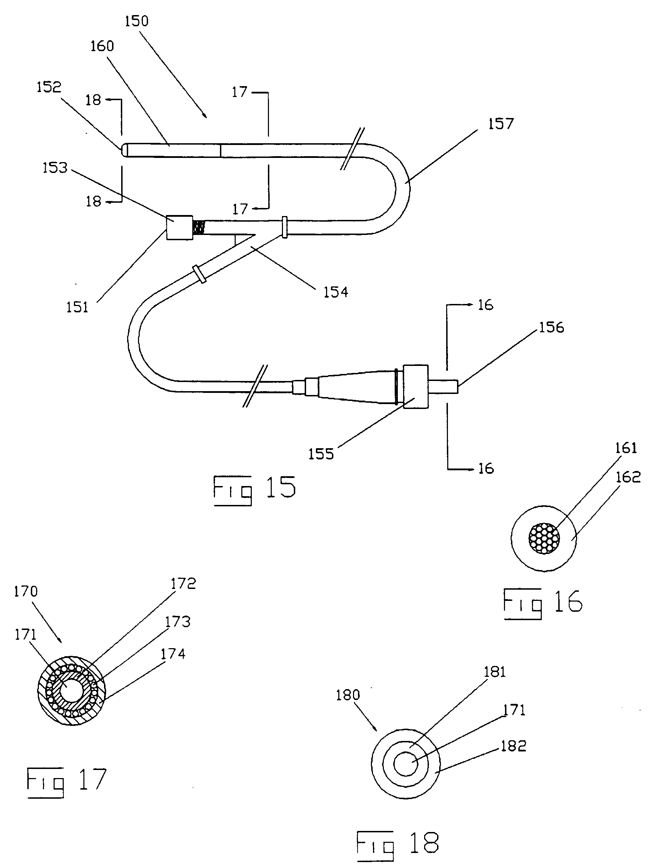

Turning now to Figure 15 a guidewire-compatible light delivery catheter useful for PDT is generally indicated at 150. The catheter has a proximal end 151, a distal end 152 and an optical connector end 156. Illuminating light enters the catheter 150 through the fiber optic bundle housed within a SMA connector 155 located at the optical connector end 156 of the catheter. The fiber optic bundle constitutes a portion of an optical waveguide which provides optical communication between the optical connector end 156 of the catheter and the distal or treatment end 152 of the catheter.

The optical connector end 156 of the catheter is shown in cross section in Figure 16 and comprises a fiber optic bundle 161 enclosed within a stainless steel connector 162. The illuminating light travels along the fiber optic bundle portion of the optical waveguide until it passes through the y adaptor 154 into the catheter body 157 where the fibers comprising the bundle are separated to surround a guidewire lumen. The guidewire lumen is a hollow conduit of a diameter sufficient to accommodate a guidewire therewithin extending between the distal end 152 of the catheter and the guidewire port 153. The guidewire lumen is indicated at 171 in the catheter cross-section shown in Figure 17 and end view of the catheter tip in Figure 18.

As mentioned previously, after it passes the y adapter 154, the fiber bundle is opened to surround and track the guidewire lumen as the optical waveguide continues along the body 157 of the catheter. The spatial relationship between the fiber bundle and the guidewire lumen within the main body of the catheter is shown in Figure 17. The guidewire lumen 171 is surrounded by an inner tubing 172 through which the guidewire may pass. The inner tubing 172 is, in turn, surrounded by the (now divided) optical fiber array. The entire catheter body 157 is finally enclosed in a durable jacket or outer tubing 174.

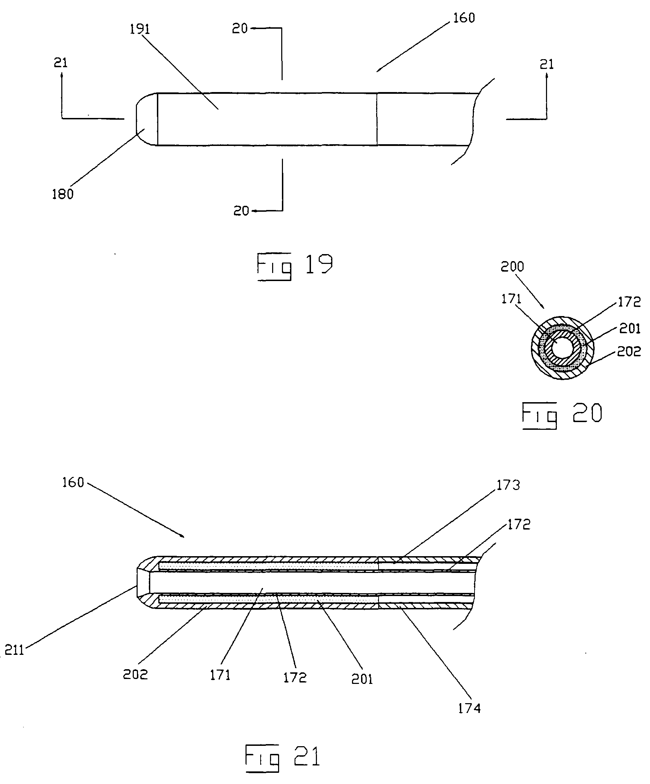

As the illuminating light continues along the optical waveguide through the catheter body 157 it reaches the diffuser tip 160. The diffuser tip, generally indicated at 160 in Figure 19, comprises a cylindrical diffuser portion 191 and a cap portion 180. The cap is enclosed with a radiopaque and light-opaque outer rounded plastic covering which facilitates passage of the catheter through a vessel and facilitates fluoroscopic placement of the tip.

The construction of the diffuser tip 160 is shown in cross section in Figure 20 and longitudinal cross section in Figure 21. The cylindrical diffuser 160 comprises a central guide wire lumen 171 surrounded by an inner tubing 172. The inner tubing is enveloped in a layer of a diffusing material 201 such as doped silicone which diffuser material 201 is, in turn, enclosed within an outer jacket of optically clear plastic tubing 202. The distal end 211 of the cylindrical diffuser comprises the cap which is shown in cross section in Figure 18.

Light conducted along the optical fiber array 173 enters the transparent diffusing material 201 where it is scattered laterally by embedded scattering centers thereafter to exit the cylindrical diffuser through the transparent jacket 202 and enter the surrounding target tissue.

Conversely, light emitted by surrounding target tissue may enter the cylindrical diffuser tip through the transparent jacket 202 whence it will encounter scattering centers and be deflected into the fiber optic array 173 of the catheter body 157 where it will be conducted to the optical connector end 156 and detected. Thus, a single catheter may be used to both detect and treat atheromatous plaque.

The foregoing embodiments of the optical fiber cylindrical diffuser tip are offered by means of example. The invention should not be limited to the specific embodiments presented herein but only by the scope of the claims appended hereto.

## Claims

1. A diffuser tip for an optical fiber comprising,
- a cylindrical central core (14) of a substantially transparent, flexible elastomer, said elastomer containing scattering centers dispersed therein, said cylindrical central core (14) having a diameter equal to or greater than the diameter of a cylindrical cladding (12) of said optical fiber, said cladding (12) immediately and concentrically overlying an elongate, cylindrical, optically transparent core (11) of said optical fiber, and
- a layer (17) of a transparent plastic concentrically surrounding said cylindrical central core (14) and at least a portion of a cylindrical outer sheath (15) of said optical fiber, said sheath (15) immediately and concentrically overlying said cladding (12) of said optical fiber,
**characterized** in that
- said cylindrical central core (14) has a diameter less than the diameter of said sheath (15) of said optical fiber,
- an intermediate layer (16) concentrically surrounds said cylindrical central core (14) and is of said elastomer and contains scattering centers, and
- said layer (17) of a transparent plastic concentrically surrounds said intermediate layer (16) and said sheath (15) of said optical fiber, said layer (17) of a transparent plastic provides a means for controlling the strength and flexibility of said diffuser tip.

2. The diffuser tip according to claim 1, **characterized** in that the concentration of said scattering centers dispersed in said elastomer of said core (14, 51, 61) increases in a distal direction along the length of said core (14, 51, 61).

3. The diffuser tip according to any of the preceding claims, **characterized** in that said elastomer is silicone.

4. The diffuser tip according to any of the preceding claims, **characterized** in that said elastomer is polyurethane.

5. The diffuser tip according to any of the preceding claims, **characterized** in that said scattering centers are selected from a group comprising alumina, Ti 02, silica or diamond dust.

## Patentansprüche

1. Diffuserspitze für eine optische Faser, aufweisend
- einen zylindrischen zentralen Kern (14) aus einem im wesentlichen transparenten, flexiblen Elastomer, wobei das Elastomer darin verteilte Streuzentren enthält, wobei der zylindrische zentrale Kern (14) einen Durchmesser hat, der gleich oder größer ist als der Durchmesser einer zylindrischen Ummantelung (12) der optischen Faser, wobei die Ummantelung (12) unmittelbar und konzentrisch über einem langgestreckten, zylindrischen, optisch transparenten Kern (11) der optischen Faser liegt, und
- eine Schicht (17) aus einem transparenten Kunststoff, die den zylindrischen zentralen Kern (14) und mindestens einen Teil einer zylindrischen äußeren Hülle (15) der optischen Faser konzentrisch umgibt, wobei die Hülle (15) unmittelbar und konzentrisch über der Ummantelung (12) der optischen Faser liegt,
**dadurch gekennzeichnet**, daß
- der zylindrische zentrale Kern (14) einen Durchmesser hat, der kleiner ist als der Durchmesser der Hülle (15) der optischen Faser,
- eine Zwischenschicht (16) den zylindrischen zentralen Kern (14) konzentrisch umgibt, wobei sie aus dem Elastomer gebildet ist und Streuzentren enthält, und
- die Schicht (17) aus einem transparenten Kunststoff die Zwischenschicht (16) und die Hülle (15) der optischen Faser konzentrisch umgibt, wobei die Schicht (17) aus einem transparenten Kunststoff eine Einrichtung zum Regeln der Stärke und der Flexibilität der Diffuserspitze bildet.

2. Diffuserspitze nach Anspruch 1, **dadurch gekennzeichnet**, daß die Konzentration der in dem Elastomer des Kerns (14, 51, 61) verteilten Streuzentren in einer distalen Richtung entlang der Länge des Kerns (14, 51, 61) ansteigt.

3. Diffuserspitze nach einem der vorhergehenden Ansprüche, **dadurch gekennzeichnet**, daß das Elastomer Silikon ist.

4. Diffuserspitze nach einem der vorhergehenden Ansprüche, **dadurch gekennzeichnet**, daß das Elastomer Polyurethan ist.

5. Diffuserspitze nach einem der vorhergehenden Ansprüche, **dadurch gekennzeichnet**, daß die Streuzentren aus einer Gruppe ausgewählt sind, die Aluminiumoxid, Ti O2, Siliziumdioxid oder Diamantstaub umfaßt.

## Revendications

1. Tête de diffusion pour une fibre optique, comprenant,
- un noyau central (14) cylindrique en un élastomère substantiellement transparent, flexible, ledit élastomère contenant, dispersés en son sein, des centres de dispersion, ledit noyau central (14) cylindrique ayant un diamètre égal ou supérieur au diamètre d'un plaquage (12) cylindrique de ladite fibre optique, ledit plaquage (12) chevauchant directement et concentriquement un noyau (11) optiquement transparent, cylindrique, allongé de ladite fibre optique, et
- une couche (17) d'une matière plastique transparente entourant concentriquement ledit noyau central (14) cylindrique et au moins une partie d'une gaine extérieure (15) cylindrique de ladite fibre optique, ladite gaine (15) chevauchant directement et concentriquement ledit plaquage (12) de ladite fibre optique,
**caractérisé** en ce que
- ledit noyau central (14) cylindrique présente un diamètre inférieur au diamètre de ladite gaine (15) de ladite fibre optique,
- une couche intermédiaire (16) entoure concentriquement ledit noyau central (14) cylindrique et est constituée dudit élastomère et contient des centres de dispersion, et
- ladite couche (17), constituée d'une matière plastique transparente, entoure concentriquement ladite couche intermédiaire (16) et ladite gaine (15) de ladite fibre optique, ladite couche (17) en matière plastique transparente fournissant un moyen permettant de contrôler la résistance mécanique et la flexibilité de ladite tête de diffusion.

2. La tête de diffusion selon la revendication 1, **caractérisée** en ce que la concentration desdits centres de dispersion dispersés dans ledit élastomère dudit noyau (14, 51, 61) augmente en direction distale le long de la longueur dudit noyau (14, 51, 61).

3. La tête de diffusion selon l'une quelconque des revendications précédentes, **caractérisée** en ce que ledit élastomère est du silicone.

4. La tête de diffusion selon l'une quelconque des revendications précédentes, **caractérisée** en ce que ledit élastomère est du polyuréthane.

5. La tête de diffusion selon l'une quelconque des revendications précédentes, **caractérisée** en ce que lesdits centres de dispersion sont sélectionnés dans un groupe comprenant l'alumine, Ti02, la silice ou la poussière de diamant.
